(19) Europäisches Patentamt
European Patent Office
Office européen des brevets

(11) Veröffentlichungsnummer: **0 024 669**
**B1**

# (12) EUROPÄISCHE PATENTSCHRIFT

(45) Veröffentlichungstag der Patentschrift: **06.04.83**

(51) Int. Cl.³: **C 07 C 103/84,** C 07 D 265/22, A 01 N 37/46, A 01 N 43/86

(21) Anmeldenummer: **80104904.0**

(22) Anmeldetag: **18.08.80**

(54) **Substituierte N-Benzoylanthranilsäurederivate und deren Anhydroverbindungen, Verfahren zu ihrer Herstellung und ihre Verwendung als Herbizide und Mittel dafür.**

(30) Priorität: **27.08.79 DE 2934543**

(43) Veröffentlichungstag der Anmeldung: **11.03.81 Patentblatt 81/10**

(45) Bekanntmachung des Hinweises auf die Patenterteilung: **06.04.83 Patentblatt 83/14**

(84) Benannte Vertragsstaaten: **AT BE CH DE FR GB IT LI NL SE**

(56) Entgegenhaltungen:
**DE-A-2 720 427**
**DE-B-1 191 171**
**FR-A-2 377 768**
**FR-A-2 412 532**
**US-A-3 914 121**

(73) Patentinhaber: **BASF Aktiengesellschaft, Carl-Bosch-Strasse 38, D-6700 Ludwigshafen (DE)**

(72) Erfinder: **Parg, Adolf, Dr., Paray-le-Monial-Strasse 8, 6702 Bad Duerkheim 5 (DE)**
Erfinder: **Wuerzer, Bruno, Dr., Ruedigerstrasse 13, D-6701 Otterstadt (DE)**
Erfinder: **Hamprecht, Gerhard, Dr., Rote-Turm-Strasse 28, D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Substituierte N-Benzoylanthranilsäurederivate und deren Anhydroverbindungen, Verfahren zu ihrer Herstellung und ihre Verwendung als Herbizide und Mittel dafür

Die vorliegende Erfindung betrifft substituierte N-Benzoylanthranilsäurederivate und deren Anhydroverbindungen, Verfahren zu ihrer Herstellung, Herbizide, die diese Verbindungen als Wirkstoffe enthalten, sowie Verfahren zur Bekämpfung unerwünschten Pflanzenwuchses mit diesen Verbindungen.

Es ist bekannt, dass 3-Phenoxybenzamide herbizid wirksam sind (DE-A-2 720 427). Weiterhin werden in der DE-PS 1 191 171 und der FR-PS 1 373 264 N-Benzoylanthranilsäure und ihre Anhydroverbindung sowie am Benzoylrest substituierte Derivate der N-Benzoylanthranilsäure und entsprechende substituierte Anhydroverbindungen als herbizid wirksam beschrieben. Die Wirkungsbeispiele zeigen, dass mit der unsubstituierten N-Benzoylanthranilsäure und ihrer Anhydroverbindung bei relativ hohen Dosierungen nur eine sehr eng begrenzte Artenzahl unerwünschter Pflanzen bekämpft werden kann. Die Verbindungen sind gut verträglich für monokotyle und dikotyle Kulturpflanzen. Ausserdem ist bekannt, dass N-Benzoylanthranilsäurederivate mit halogen- oder alkoxysubstituiertem Benzoylrest fungizid wirksam sind (FR-A-2 377 768).

Eine Reihe von substituierten Anhydroverbindungen, d.h. 4H-3,1-Benzoxazin-4-onen, die in 2-Stellung einen substituierten Phenylrest tragen, werden in der US-PS 3 914 121 und der US-PS 3 970 652 als nicht-phytotoxisch bzw. aufgrund der erforderlichen hohen aufwandmenge als wertlose Herbizide beschrieben.

Als besonders nützlich zur Bekämpfung von unerwünschter Vegetation in Getreide und zur Beseitigung breitblättriger Unkräuter in Sojabohnen wird in der US-PS 3 914 121 und der US-PS 3 970 652 eine Auswahl von 2-Aryl-4H-3,1-benzoxazin-4-on-Derivaten hervorgehoben, beispielsweise das 2-(m-Trifluormethyl-phenyl)-4H-3,1-benzoxazin-4-on. Dabei fällt auf, dass mit einer hohen Aufwandmenge von rund 11 kg/ha gearbeitet wird. Es wird zwar eine breite Palette monokotyler und dikotyler Kulturpflanzen als Indikatoren für die Phytotoxizität der Verbindungen aufgeführt, typische Vertreter von in Äckern verbreiteten breitblättrigen Unkräutern fehlen allerdings.

Es wurde gefunden, dass substituierte N-Benzoylanthranilsäurederivate der Formel I:

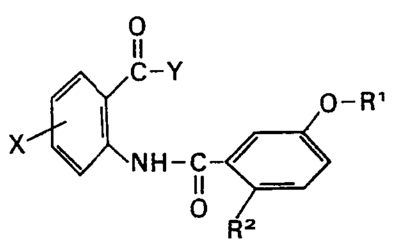

(I)

und deren Anhydroverbindungen der Formel II:

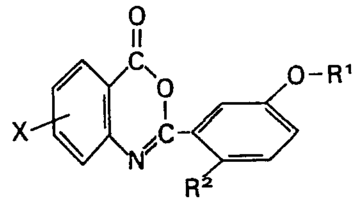

(II)

in denen

$R^1$ für einen substituierten Phenylrest der Formel:

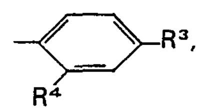

wobei $R^3$ und $R^4$ jeweils unabhängig voneinander Halogen oder Halogenalkyl mit 1 bis 4 Kohlenstoffatomen bedeuten,

$R^2$ für Wasserstoff oder Nitro,

X für Wasserstoff, Halogen, Alkyl oder Alkoxy mit jeweils 1 bis 4 Kohlenstoffatomen und Y für den Rest $-OR^6$,

in dem $R^6$ Wasserstoff, Alkyl mit 1 bis 4 Kohlenstoffatomen oder ein Alkalimetallkation bedeutet, stehen,

bei verhältnismässig niedrigen Dosierungen selektiv herbizid wirken.

$R^1$ in den Formeln I und II kann für einen substituierten Phenylrest der Formel:

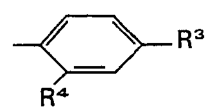

stehen,

wobei $R^3$ und $R^4$ jeweils unabhängig voneinander beispielsweise Fluor, Chlor, Brom, Jod, Trifluormethyl, Difluormethyl, Fluormethyl, Trichlormethyl, Dichlormethyl, Chlormethyl, Difluorchlormethyl, 1-Chloräthyl, 2-Chloräthyl, 1-Fluoräthyl, 2-Fluoräthyl, 2,2,2-Trichloräthyl, 2,2,2-Trifluoräthyl, 1,1,2,2-Tetrafluoräthyl, 1,1,2-Trifluor-2-chloräthyl, 1,1,2,2,2-Pentafluoräthyl bedeuten.

X kann beispielsweise Wasserstoff, Fluor, Chlor, Brom, Jod, Methyl, Äthyl, n-Propyl, i-Propyl, n-Butyl, tert.-Butyl, Methoxy, Äthoxy bedeuten.

Y steht für den Rest $-O-R^6$, in dem $R^6$ beispielsweise Wasserstoff, Methyl, Äthyl, n-Propyl, i-Propyl, n-Butyl, sek.-Butyl, Isobutyl, tert.-Butyl, ein Lithium-, Kalium-, Natriumion bedeutet.

Bevorzugte Verbindungen sind solche, bei denen $R^1$ einen durch Chlor und Trifluormethyl substituierten Phenylrest, $R^2$ Wasserstoff oder Nitro, X Wasserstoff, Chlor, Methyl oder Methoxy und Y, wenn es sich um Verbindungen der Formel I handelt,

den Rest $-OR^6$ bedeutet, wobei $R^6$ Wasserstoff, Methyl oder ein Alkalimetallkation bedeutet.

Die N-Benzoylanthranilsäurederivate der Formel I erhält man durch Umsetzung von Anthranilsäurederivaten der Formel III:

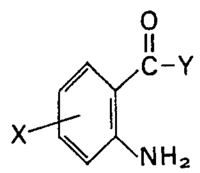

(III)

in der X und Y die obengenannten Bedeutungen haben, mit ungefähr stöchiometrischen Mengen eines substituierten Benzoylhalogenids der Formel IV:

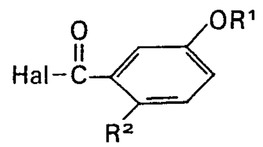

(IV)

in der $R^1$ und $R^2$ die obengenannten Bedeutungen haben und Hal für Halogen, insbesondere Fluor, Chlor oder Brom, steht, in wässrigem alkalischem Medium oder gegebenenfalls in Gegenwart eines säurebindenden Mittels in einem inerten organischen Lösungsmittel bei einer Temperatur im Bereich zwischen −30 und +150°C.

Zweckmässigerweise wird das Anthranilsäurederivat der Formel III dabei in wässerigem alkalischem Medium mit der ungefähr äquimolaren Menge an Benzoylhalogenid der Formel IV und der mindestens äquimolaren Menge an Alkalihydroxid, bezogen auf beide Ausgangsstoffe, umgesetzt [„J. Org. Chem.", *9*, 396-400 (1944)]. Ebenso kann die Reaktion gegebenenfalls in Anwesenheit eines säurebindenden Mittels in einem inerten organischen Lösungsmittel durchgeführt werden. Bei beiden Verfahrensweisen kann die Reaktionstemperatur im Bereich zwischen −30 und +150°C, vorzugsweise zwischen +20 und +80°C, variiert werden.

Die Umsetzung kann sowohl diskontinuierlich als auch kontinuierlich durchgeführt werden.

Als inerte organische Lösungsmittel eignen sich Kohlenwasserstoffe, wie Ligroin, Benzin, Toluol, Pentan, Hexan, Cyclohexan, Petroläther; aliphatische und aromatische Halogenkohlenwasserstoffe, wie Methylenchlorid, Chloroform, Tetrachlorkohlenstoff, 1,1- und 1,2-Dichloräthan, 1,1,1- und 1,1,2-Trichloräthan, Chlorbenzol, o,m,p-Dichlorbenzol, o,m,p-Chlortoluol; aliphatische und aromatische Nitrokohlenwasserstoffe, wie Nitrobenzol, Nitroäthan, o,m,p-Nitrotoluol; Nitrile, wie Acetonitril, Butyronitril, Isobutyronitril; Äther, wie Diäthyläther, Di-n-propyläther, Tetrahydrofuran, Dioxan; Ester, wie Acetessigester, Äthylacetat oder Isobutylacetat; und Amide, wie Formamid, Methylformamid und Dimethylformamid.

Als säurebindende Mittel können beispielsweise Alkalihydroxide, Alkalicarbonate und tertiäre organische Basen verwendet werden. Besonders geeignet sind Natriumhydroxid, Natriumcarbonat, Natriumhydrogencarbonat, Triäthylamin, Pyridin, Trimethylamin, α-, β-, γ-Picolin, Lutidine, N,N-Dimethylanilin, N,N-Dimethylcyclohexylamin, Chinolin, Tri-n-propylamin, Tri-n-butylamin, Acridin.

Die Zugabe der Ausgangsstoffe I und II kann in beliebiger Reihenfolge erfolgen.

Die Verbindungen der Formel I können aus der Reaktionsmischung isoliert werden, indem zunächst der gebildete Niederschlag abgesaugt und mit Wasser verührt wird, wonach der verbleibende Rückstand wiederum abfiltriert wird. Ist das Endprodukt im Lösungsmittel löslich, wird dieses unter vermindertem Druck abgezogen, der Rückstand in Alkalilauge aufgenommen, filtriert und anschliessend mit Säure versetzt. Zur Isolierung des Endprodukts wird abgesaugt, der Rückstand wird umkristallisiert oder chromatographiert.

Die Anhydroverbindungen der Formel II lassen sich durch Umsetzung von Anthranilsäurederivaten der Formel III:

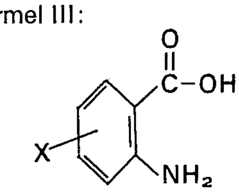

(V)

in der X die obengenannten Bedeutungen hat, mit mindestens dem einfachen molaren Überschuss eines substituierten Benzoylhalogenids der Formel IV:

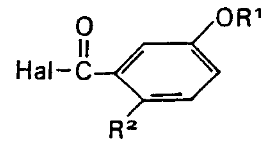

(IV)

in der $R^1$ und $R^2$ die obengenannten Bedeutungen haben und Hal für Halogen, insbesondere Fluor, Chlor oder Brom, steht, in Gegenwart eines aromatischen tertiären Amins bei einer Temperatur im Bereich zwischen 0 und 150°C herstellen.

Geeignete aromatische tertiäre Amine sind beispielsweise Pyridin, Chinolin, α-, β-, γ-Picolin, Acridin, α- und γ-Lutidin. Man verwendet die 2- bis 10-fache molare Menge an Amin pro Mol Anthranilsäurederivat der Formel V.

Zur Umsetzung wird das Benzoylhalogenid zweckmässigerweise in die Lösung des Anthranilsäurederivats im Amin einlaufen lassen. Die Umsetzung kann dabei kontinuierlich oder diskontinuierlich durchgeführt werden. Als Reaktionstemperatur wählt man eine Temperatur im Bereich zwischen 0 und 150°C, vorzugsweise 20 und 80°C („J. Chem. Soc." (c) 1968, S. 1593-1597).

Die Verbindungen der Formel II können durch Einrühren des Reaktionsgemisches in Wasser isoliert werden. Dabei wird der verbleibende Rückstand abgesaugt, gegebenenfalls durch eine Alkaliwäsche von Spuren saurer Verunreinigung befreit und anschliessend durch Umkristallisieren oder Chromatographieren gereinigt.

Anhydroverbindungen der Formel II können auch durch Cyclisierung von N-Benzoyl-anthranilsäurederivat der Formel I in Gegenwart eines wasserentziehenden Mittels bei einer Temperatur zwischen 0 und 150°C erhalten werden.

Zu den bevorzugten wasserentziehenden Mitteln gehören Carbonsäureanhydride, wie Essigsäureanhydrid, Propionsäureanhydrid, Buttersäureanhydrid, gemischte Carbonsäureanhydride, wie Ameisensäureessigsäureanhydrid, Ameisensäurepropionsäureanhydrid, Ameisensäurebuttersäureanhydrid, Essigsäurepropionsäureanhydrid,

Essigsäurebuttersäureanhydrid, Propionsäurebuttersäureanhydrid, ferner Dicyclohexylcarbodiimid und Thionylchlorid. Die Cyclisierung wird unter Zusatz der 1- bis 10-fachen molaren Menge an wasserentziehendem Mittel, bezogen auf N-Benzoyl-anthranilsäurederivat der Formel I, durchgeführt.

Die Reaktion kann bei einer Temperatur von 0 bis 150°C, vorzugsweise von 50 bis 100°C, während 30 min bis 5 h kontinuierlich oder diskontinuierlich geführt werden.

Die Isolierung der Endprodukte aus der Reaktionslösung erfolgt durch Einengen des Reaktionsgemisches bis zur Trockne. Der verbleibende Rückstand kann gegebenenfalls durch eine Alkaliwäsche von Spuren saurer Verunreinigung befreit und anschliessend durch Umkristallisation oder Chromatographieren gereinigt werden [„J. Org. Chem." *14*, 967-981 (1949)].

Die als Ausgangsstoffe benötigten Benzoylhalogenide der Formel IV können nach bekannten Methoden aus den entsprechenden Benzoesäuren hergestellt werden. (Houben-Weyl, „Methoden der organ. Chem.", Bd. 8, S. 463 ff., Georg Thieme-Verlag, Stuttgart, 1952.) Die Phenoxy- bzw. Heteroaryloxy-substituierten Benzoesäuren können nach der Methode der Williamson'schen Äthersynthese aus dem Phenolat der jeweiligen 3-Hydroxybenzoesäure und den entsprechenden Halogenbenzolen erhalten werden.

Die folgenden Beispiele erläutern die Herstellung der erfindungsgemässen Verbindungen der Formeln I und II. Gewichtsteile verhalten sich zu Volumenteilen wie kg zu l.

*Beispiel 1*

Zu einer Lösung von 75,58 Gewichtsteilen Anthranilsäuremethylester in 500 Volumenteilen absolutem Diäthyläther werden bei Raumtemperatur gleichzeitig eine Lösung von 190 Volumenteilen 3-(2'-Chlor-4'-trifluormethylphenoxy)-6-nitrobenzoesäurechlorid in 120 Volumenteilen absolutem Diäthyläther und 50,5 Gewichtsteile Triäthylamin zugetropft. Die Reaktionsmischung wird anschliessend auf Rückfluss erhitzt, 1 h nachgerührt, abgekühlt und abgesaugt. Der Rückstand wird in 2000 Gewichtsteile Wasser eingerührt und auschliessend abgesaugt. Man erhält 240 Gewichtsteile (97% d.Th.) N-3'-(2"-Chlor-4"-trifluormethylphenoxy)-6'-nitrobenzoylanthranilsäuremethylester vom Fp. 123-126°C.

*Analyse:*

berechnet: C 52,5 H 2,5 N 5,8 Cl 7,4 F 11,85%
gefunden: C 52,6 H 2,8 N 5,8 Cl 7,4 F 11,4 %

*Beispiel 2*

247 Gewichtsteile N-3'-(2"-Chlor-4"-trifluormethylphenoxy)-6'-nitrobenzoylanthranilsäuremethylester werden zusammen mit einer Lösung von 33,7 Gewichtsteilen Kaliumhydroxid in 200 Gewichtsteilen Wasser und 200 Volumenteilen Äthanol 1 h unter Rückfluss erhitzt. Danach kühlt man ab und säuert die klare Reaktionslösung mit 3 n Salzsäure an. Dabei scheidet sich ein Öl ab, das in Diäthyläther aufgenommen wird. Durch Zusatz von Petroläther erhält man ein kristallines Produkt; nach dem Absaugen erhält man 200 Gewichtsteile (83% d.Th.) N-3'-(2"-Chlor-4"-trifluormethylphenoxy)-6'-nitrobenzoylanthranilsäure vom Fp. 187-192°C.

*Analyse:*

berechnet: C 52,5 H 2,5 N 5,8 Cl 7,4 F 11,85%
gefunden: C 52,6 H 2,8 N 5,8 Cl 7,4 F 11,4 %

*Beispiel 3*

Zu einer Lösung von 6,9 Gewichtsteilen Anthranilsäure in 100 Volumenteilen absolutem Tetrahydrofuran werden bei Raumtemperatur 18,1 Gewichtsteile 3-(2'-Chlor-4'-trifluormethylphenoxy)-6-nitrobenzoesäurechlorid in 40 Volumenteilen absolutem Tetrahydrofuran und 5,1 Gewichtsteile Triäthylamin gleichzeitig zugetropft. Die Reaktionsmischung wird anschliessend auf Rückfluss aufgeheizt und 1 h nachgerührt. Nach dem Abkühlen saugt man ab und engt das Filtrat ein. Der ölige Rückstand wird in 40 Volumenteilen Diäthyläther aufgenommen, einmal mit verdünnter Salzsäure und dann zweimal mit 1 n Natronlauge extrahiert. Die vereinigten alkalischen Phasen werden dann mit 2 n Salzsäure angesäuert und das Produkt abgesaugt. Man erhält 10 Gewichtsteile (42% d.Th.) N-3'-(2"-Chlor-4"-trifluormethylphenoxy)-6'-nitrobenzoylanthranilsäure vom Fp. 182-185°C.

*Analyse:*

berechnet: C 52,5 H 2,5 N 5,8 Cl 7,8 F 11,85%
gefunden: C 52,2 H 2,2 N 5,9 Cl 7,2 F 10,9 %

*Beispiel 4*

24 Gewichtsteile N-3'-(2"-Chlor-4"-trifluormethylphenoxy)-6-nitrobenzoylanthranilsäure werden in eine Lösung von 2 Gewichtsteile Natriumhydroxid in 100 Volumenteilen absolutem Methanol eingetragen und 1 h bei Raumtemperatur nachgerührt. Nach Einengen der Lösung verbleiben als Rückstand 25 Gewichtsteile (100% d.Th.) N-3'-(2"-Chlor-4"-trifluormethyl-phenoxy)-6'-nitrobenzoylanthranilsäurenatriumsalz.

*Analyse:*

berechnet: C 50,2 H 2,2 N 5,6 Cl 7,1 Na 4,6%
gefunden: C 49,9 H 2,6 N 5,6 Cl 7,0 Na 4,0%

*Beispiel 5*

24 Teile N-3-(2'-Chlor-4'-trifluormethyl)phenoxy-6-nitrobenzoylanthranilsäure werden mit 200 Teilen Essigsäureanhydrid 4 h auf Rückfluss erhitzt. Man filtriert ab und engt im Vakuum zur Trockne ein. Nach Umkristallisation des Rückstandes aus Äthanol erhält man 18 Teile (78% d.Th.) 2-[3'-(2"-Chlor-4"trifluormethyl-phenoxy)-6'-nitrophenyl]-4H-1,3-benzoxazin-4-on vom Fp. 131-135°C.

*Analyse:*

berechnet:
C 54,51 H 2,18 N 6,05 Cl 7,66 F 12,32%
gefunden:
C 54,2 H 2,3 N 5,9 Cl 7,2 F 12,0 %

Nach analogen Verfahren können beispielsweise folgende Verbindungen der Formeln I und II synthetisiert werden:

a) Verbindungen der Formel I:

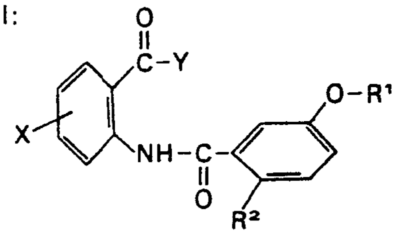

(I)

| Nr. | $R^1$ | $R^2$ | X | Y | Fp. [°C] |
|---|---|---|---|---|---|
| 6 | 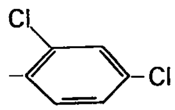 | H | H | OH | |
| 7 | 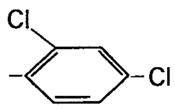 | $NO_2$ | H | OH | 125-130 |
| 8 | 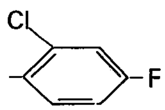 | H | H | OH | |
| 9 | 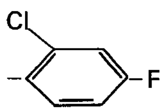 | $NO_2$ | H | OH | |
| 10 | 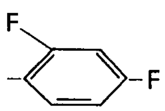 | H | H | OH | |
| 11 | 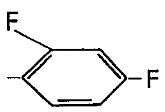 | $NO_2$ | H | OH | |
| 12 | 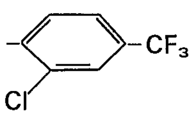 | H | H | OH | 178-183 |
| 13 | 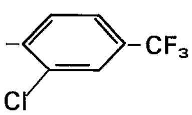 | H | H | $OCH_3$ | 88-92 |
| 14 | 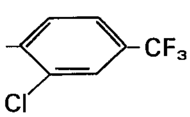 | H | 3-$OCH_3$ | OH | |
| 15 | 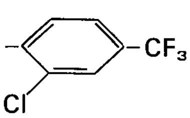 | $NO_2$ | 3-$OCH_3$ | OH | 185-192 |

| Nr. | $R^1$ | $R^2$ | X | Y | Fp. [°C] |
|---|---|---|---|---|---|
| 16 | 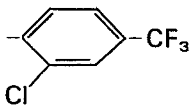 | $NO_2$ | 6-Cl | OH | 130-135 |
| 17 | 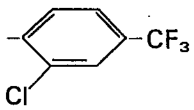 | $NO_2$ | 6-F | OH | 162-164 |
| 18 | 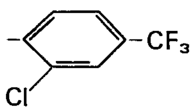 | H | $3\text{-}CH_3$ | OH | 156-159 |
| 19 | 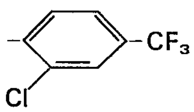 | H | $3\text{-}CH_3$ | $OCH_3$ | 83-87 |
| 20 | 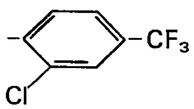 | H | $3\text{-}CH_3$ | ONa | $[\nu_{C=O} = 1580\ cm^{-1}]$ |
| 21 | 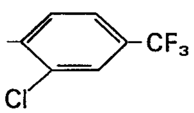 | $NO_2$ | $3\text{-}CH_3$ | OH | 216-219 |
| 22 | 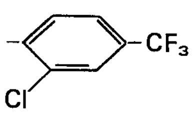 | H | 3-Cl | OH | 120-126 |
| 23 | 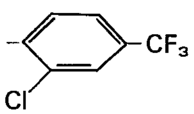 | $NO_2$ | 3-Cl | OH | 209-211 |
| 24 | 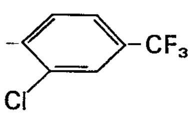 | $NO_2$ | 3-Cl | $OCH_3$ | 140-143 |
| 25 | 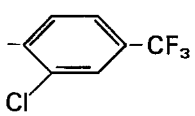 | H | 3-F | OH | |
| 26 | 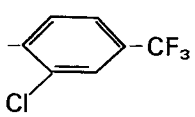 | $NO_2$ | 3-F | OH | |
| 27 | 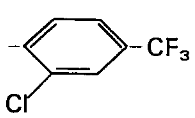 | H | 4-Cl | OH | |
| 28 | 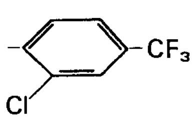 | $NO_2$ | 4-Cl | $OCH_3$ | 186-189 |

| Nr. | $R^1$ | $R^2$ | X | Y | Fp. [°C] |
|---|---|---|---|---|---|
| 29 | 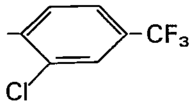 | $NO_2$ | 4-Cl | OH | 230-232 |
| 30 | 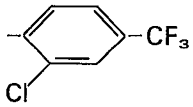 | $NO_2$ | 4-Cl | ONa | [$\nu_{C=O}$ = 1670 $cm^{-1}$] |
| 31 | 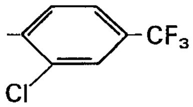 | CN | H | OH | |
| 32 | 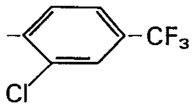 | H | H | $OCH_3$ | 100-104 |
| 33 | 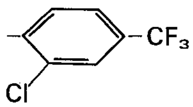 | H | H | ONa | [$\nu_{C=O}$ = 1680 $cm^{-1}$] |
| 34 | 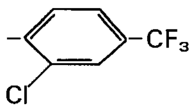 | $NO_2$ | H | $OCH_3$ | 85-92 |
| 35 | 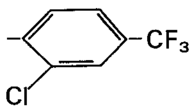 | $NO_2$ | H | ONa | [$\nu_{C=O}$ = 1660 $cm^{-1}$] |
| 36 | 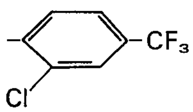 | $NO_2$ | 5-Cl | OH | 223-227 |
| 37 | 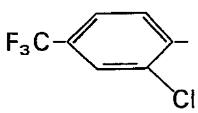 | $NO_2$ | 3-$CH_3$ | $OCH_3$ | 170-173 |
| 38 | 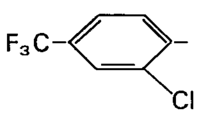 | $NO_2$ | 3-$OCH_3$ | $OCH_3$ | 150-154 |
| 39 | 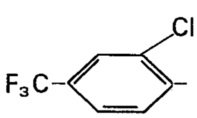 | $NO_2$ | 5-Cl | OH | 223-227 |
| 40 | 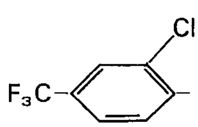 | H | H | ONa | [$\nu_{C=O}$ = 1580 $cm^{-1}$] |
| 41 | 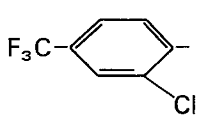 | H | 6-Cl | $OCH_3$ | $n_D^{25}$ = 1,5858 |

| Nr. | $R^1$ | $R^2$ | X | Y | Fp. [°C] |
|---|---|---|---|---|---|
| 42 | 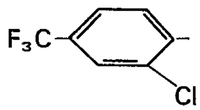 | H | 6-Cl | OH | 181-185 |
| 43 | 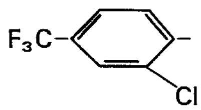 | H | 6-Cl | ONa | $[\nu_{C=O} = 1600\ cm^{-1}]$ |

b) Verbindungen der Formel II

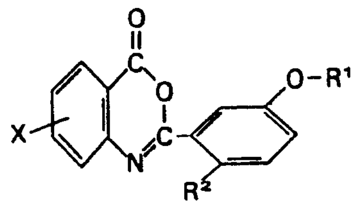

(II)

| Nr. | $R^1$ | $R^2$ | X | Fp. [°C] |
|---|---|---|---|---|
| 44 | 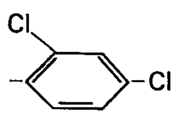 | H | H | |
| 45 | 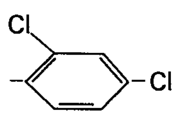 | $NO_2$ | H | 165-170 |
| 46 | 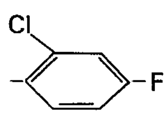 | H | H | |
| 47 | 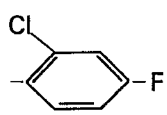 | $NO_2$ | H | |
| 48 | 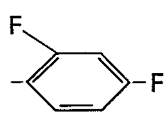 | $NO_2$ | H | |
| 49 | 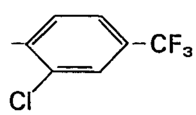 | H | H | 141-143 |
| 50 | 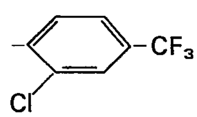 | $NO_2$ | 8-$OCH_3$ | 175-178 |
| 51 | 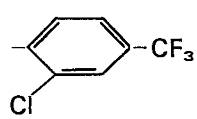 | $NO_2$ | 8-$CH_3$ | 129-134 |

| Nr. | $R^1$ | $R^2$ | X | Fp. [°C] |
|---|---|---|---|---|
| 52 | 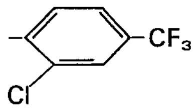 | H | 8-$CH_3$ | 83-87 |
| 53 | 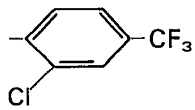 | $NO_2$ | 5-Cl | 161-164 |
| 54 | 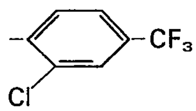 | $NO_2$ | 5-F | 109-113 |
| 55 | 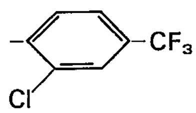 | $NO_2$ | 7-Cl | 138-141 |
| 56 | 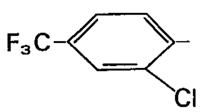 | H | 8-Cl | 120-128 |
| 57 | 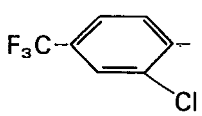 | H | 5-Cl | 150-153 |

In Anbetracht der guten Verträglichkeit und der Vielseitigkeit der Applikationsmethoden können die erfindungsgemässen Herbizide oder diese enthaltende Mittel in einer grossen Zahl von Kulturpflanzen zur Beseitigung unerwünschten Pflanzenwuchses eingesetzt werden. Die Aufwandmengen können dabei je nach Zusammensetzung und Wachstummsstadien der Unkrautflora zwischen 0,1 und 15, vorzugsweise jedoch zwischen 0,2 und 3,0 kg pro Hektar schwanken.

In Betracht kommen beispielsweise folgende Kulturen:

| Botanischer Name (Abk. in Tabelle) | Deutscher Name | Englischer Name |
|---|---|---|
| *Allium cepa* | Küchenzwiebel | onions |
| *Ananas comosus* | Ananas | pineapple |
| *Arachis hypogaea* | Erdnuss | peanuts (groundnuts) |
| *Asparagus officinalis* | Spargel | asparagus |
| *Avena sativa* | Hafer | oats |
| *Beta vulgaris spp. altissima* | Zuckerrübe | sugarbeets |
| *Beta vulgaris spp. rapa* | Futterrübe | fodder beets |
| *Beta vulgaris spp. esculenta* | Rote Rübe | table beets, red beets |
| *Brassica napus var. napus* | Raps | rape seed |
| *Brassica napus var. napobrassica* | Kohlrübe | |
| *Brassica napus var. rapa* | Weisse Rübe | turnips |
| *Brassica rapa var. silvestris* | Rübsen | |
| *Camellia sinensis* | Teestrauch | tea plants |
| *Carthamus tinctorius* | Saflor-Färberdistel | safflower |
| *Carya illinoinensis* | Pekannussbaum | pecan trees |
| *Citrus limon* | Zitrone | lemon |
| *Citrus maxima* | Pampelmuse | grapefruits |
| *Citrus reticulata* | Mandarine | |
| *Citrus sinensis* | Apfelsine, Orange | orange trees |
| *Coffea arabica (Coffea canephora, Coffea liberica)* | Kafee | coffee plants |
| *Cucumis melo* | Melone | melons |

| Botanischer Name (Abk. in Tabelle) | Deutscher Name | Englischer Name |
|---|---|---|
| *Cucumis sativus* | Gurke | cucumber |
| *Cynodon dactylon* | Bermudagras | Bermudagrass in turfs and lawns |
| *Daucus carota* | Möhre | carrots |
| *Elaeis guineensis* | Ölpalme | oil palms |
| *Fragaria vesca* | Erdbeere | strawberries |
| *Glycine max* | Sojabohne | soybeans |
| *Gossypium hirsutum (Gossypium arboreum Gossypium herbaceum Gossypium vitifolium)* | Baumwolle | cotton |
| *Helianthus annuus* | Sonnenblume | sunflowers |
| *Helianthus tuberosus* | Topinambur | |
| *Hevea brasiliensis* | Parakautschukbaum | rubber plants |
| *Hordeum vulgare* | Gerste | barley |
| *Humulus lupulus* | Hopfen | hop |
| *Ipomea batatas* | Süsskartoffeln | sweet potato |
| *Juglans regia* | Walnussbaum | walnut trees |
| *Lactua sativa* | Kopfsalat | lettuce |
| *Lens culinaris* | Linse | lentils |
| *Linum usitatissimum* | Faserlein | flax |
| *Lycopersiocon lycopersicum* | Tomate | tomato |
| *Malus spp.* | Apfel | apple trees |
| *Manihot esculenta* | Maniok | cassava |
| *Medicago sativa* | Luzerne | alfalfa (lucerne) |
| *Mentha piperita* | Pfefferminze | peppermint |
| *Musa spp.* | Obst.- u. Mehlbanane | banana plants |
| *Nicotiina tabacum (N. rustica)* | Tabak | tobacco |
| *Olea europea* | Ölbaum | olive trees |
| *Oryza sativa* | Reis | rice |
| *Panicum miliaceum* | Rispenhirse | |
| *Phaseolus lunatus* | Mondbohne | limabeans |
| *Phaseolus mungo* | Erdbohne | mungbeans |
| *Phaseolus vulgaris* | Buschbohnen | snapbeans, green beens, dry beans |
| *Pennisetum glaucum* | Perl- oder Rohrkolbenhirse | |
| *Petroselinim crispum spp. tuberosum* | Wurzelpetersilie | parsley |
| *Picea abies* | Rotfichte | Norway spruce |
| *Abies alba* | Weisstanne | fire |
| *Pinus spp.* | Kiefer | pine trees |
| *Pisum sativum* | Gartenerbse | English peas |
| *Prunus avium* | Süsskirsche | cherry trees |
| *Prunus domestica* | Pflaume | plum trees |
| *Prunus dulcis* | Mandelbaum | almond trees |
| *Prunus persica* | Pfirsich | peach trees |
| *Pyrus communis* | Birne | pear trees |
| *Ribes sylvestre* | Rote Johannisbeere | red currants |
| *Ribes uva-crispa* | Stachelbeere | |
| *Ricinus communis* | Rizinus | |
| *Saccharum officinarum* | Zuckerrohr | sugar cane |
| *Secale cereale* | Roggen | rye |
| *Sesamum indicum* | Sesam | Sesame |
| *Solanum tuberosum* | Kartoffel | Irish potato |
| *Sorghum bicolor (s. vulgare)* | Mohrenhirse | sorghum |
| *Sorghum dochna* | Zuckerhirse | |
| *Spinacia oleracea* | Spinat | spinach |
| *Theobroma cacao* | Kakaobaum | cacao plants |
| *Trifolium pratense* | Rotklee | red clover |
| *Triticum aestivum* | Weizen | wheat |
| *Vaccinium carymbosum* | Kulturheidelbeere | blueberry |
| *Vaccinium vitis-idaea* | Preiselbeere | cranberry |
| *Vicia faba* | Pferdebohnen | tick beans |

| Botanischer Name (Abk. in Tabelle) | Deutscher Name | Englischer Name |
|---|---|---|
| *Vigna sinensis (V. unguiculata)* | Kuhbohne | cow peas |
| *Vitis vinifera* | Weinrebe | grapes |
| *Zea mays* | Mais | Indian corn, sweet corn |

Die erfindungsgemässen Substanzen können beispielsweise in Form von direkt versprühbaren Lösungen, Pulvern, Suspensionen, auch hochprozentigen wässerigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt wie Kerosin oder Dieselöl, ferner Kohlenteeröle, sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Signinsulfonsäure, Naphthalinsulfonsäuren, Phenolsulfonsäure, Alkylarylsulfonate, Alkylsulfate, Alkylsulfonate, Alkali- und Erdalkalisalze der dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphtalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol-, Octylphenol-, Nonylphenol, Alkylphenyolpolyglykoläther, Tributylphenylpolyglykoläther, Alkalarylpolyätheralkohole, Isotridecylalkohol, Fettalkoholäthylenoxidkondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylzellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenderde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Zellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung gemäss Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäss Beispiel 2 werden in einer Mischung gelöst, die aus 80 Gewichtsteilen Xylol, 10 Gewichtsteilen des Anlagerungsproduktes von 8 bis 10 mol Äthylenoxid an 1 mol Ölsäure-N-monoäthanolamid, 5 Gewichtsteilen Calciumsalz der Dodecylbenzolsulfonsäure und 5 Gewichtsteilen des Anlagerungsproduktes von 40 mol Äthylenoxid an 1 mol Rizinusöl besteht. Durch Ausgiessen und feines Verteilen der Lösung in 100000 Gewichtsteilen Wasser erhält man eine wässerige Dispersion, die 0,02 Gewichtsprozent des Wirkstoffs enthält.

III. 20 Gewichtsteile der Verbindung Nr. 37 werden in einer Mischung gelöst, die aus 40 Gewichtsteilen Cyclohexanon, 30 Gewichsteilen Isobutanol, 20 Gewichtsteilen des Anlagerungsproduktes von 7 mol Äthylenoxid an 1 mol Isooctylphenol und 10 Gewichtsteilen des Anlagerungsproduktes von 40 mol Äthylenoxid an 1 mol Rizinusöl besteht. Durch Eingiessen und feines Verteilen der Lösung in 100000 Gewichtsteilen Wasser erhält man eine wässerige Dispersion, die 0,02 Gewichtsprozent des Wirkstoffs enthält.

IV. 20 Gewichtsteile der Verbindung gemäss Beispiel 5 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 mol Äthylenoxid an 1 mol Rizinusöl besteht. Durch Eingiessen und feines Verteilen der Lösung in 100000 Gewichtsteilen Wasser erhält man eine wässerige Dispersion, die 0,02 Gewichtsprozent des Wirkstoffs enthält.

V. 20 Gewichtsteile der Verbindung gemäss Beispiel 4 werden mit 3 Gewichtsteilen des Natriumsalzes der Diisobutylnaphtalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gewichtsprozent des Wirkstoffs enthält.

VI. 3 Gewichtsteile der Verbindung Nr. 20 werden mit 97 Gewichtsteilen feinteiligem Kaolin vermischt. Man erhält auf diese Weise ein Stäubemittel, das 3 Gewichtsprozent des Wirkstoffs enthält.

VIII. 20 Teile der Verbindung Nr. 11 werden mit 2 Teilen Calciumsalz der Dodecylbenzolsulfonsäure, 8 Teilen Fettalkoholpolyglykoläther, 2 Teilen Natriumsalz eines Phenolharnstoffformaldehydkondensates und 68 Teilen eines paraffinischen Mineralöls innig vermischt. Man erhält eine stabile ölige Dispersion.

Die N-Benzoylanthranilsäurederivate der Formel I und ihre Anhydroverbindungen der Formel II können sowohl unter sich als auch mit zahhlreichen Vertretern anderer herbizider oder wachstumsregulierender Wirkstoffe gemischt und gemeinsam ausgebracht werden. Beispielsweise kommen als Mischungskomponenten Diazine, Benzothiadiazinone, 2,6-Dinitroaniline, N-Phenylcarbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreiterung des Wirkungsspektrums und erzielen zuweilen synergistische Effekte.

Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon
5-Methylamino-4-chlor-2-m-trifluormethylphenyl-3(2H)-pyridazinon
5-Methylamino-4-chlor-2-m-α,α,β,β-tetrafluoräthoxyphenyl-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-m-trifluormethylphenyl-3(2H)-pyridazinon
5-Methoxy-4-chlor-2-m-trifluormethylphenyl-3(2H)-pyridazinon
5-Amino-4-brom-2-m-methylphenyl-3(2H)-pyridazinon
3-(1-Methyläthyl)-1H-2,1,3-benzothiadiazin-4(3H)-on-2,2-dioxid und Salze
3-(1-Methyläthyl)-8-chlor-1H-2,1,3-benzothiadiazin-4(3H)-on-2,2-dioxid und Salze
3-(1-Methyläthyl)-8-fluor-1H-2,1,3-benzothiadiazin-4(3H)-on-2,2-dioxid und Salze
3-(1-Methyläthyl)-8-methyl-1H-2,1,3-benzothiadiazin-4(3H)-on-2,2-dioxid und Salze
1-Methoxymethyl-3-(1-methyläthyl)-2,1,3-benzothiadizin-4(3H)-on-2,2-dioxid
1-Methoxymethyl-8-chlor-3-(1-methyläthyl)-2,1,3-benzothiadiazin-4(3H)-on-2,2-dioxid
1-Methoxymethyl-8-fluor-3-(1-methyläthyl)-2,1,3-benzothiadiazin-4(3H)-on-2,2-dioxid
1-Cyan-8-chlor-3-(1-methyläthyl)-2,1,3-benzothiadiazin-4(3H)-on-2,2-dioxid
1-Cyan-8-fluor-3-(1-methyläthyl)-2,1,3-benzothiadiazin-4(3H)-on-2,2-dioxid
1-Cyan-8-methyl-3-(1-methyläthyl)-2,1,3-benzothiadizin-4(3H)-on-2,2-dioxid
1-Cyan-3-(1-methyläthyl)-2,1,3-benzothiadiazin-4(3H)-on-2,2-dioxid
1-Azidomethyl-3-(1-methyläthyl)-2,1,3-benzothiadiazin-4(3H)-on-2,2-dioxid
3-(1-methyläthyl)-1H-(pyridino)-[3,2-e]2,1,3-thiadiazin-(4)-on-2,2-dioxid
N-(1-Äthylpropyl)-2,6-dinitro-3,4-dimethylanilin
N-(1-Methyläthyl)-N-äthyl-2,6-dinitro-4-trifluormethylanilin
N-n-Propyl-N-β-chloräthyl-2,6-dinitro-4-trifluormethylanilin
N-n-Propyl-N-cyclopropylmethyl-2,6-dinitro-4-trifluormethylanilin
N-Bis(n-propyl)-2,6-dinitro-3-amino-4-trifluormethylanilin
N-Bis(n-propyl)-2,6-dinitro-4-methyl-anilin
N-Bis(n-propyl)-2,6-dinitro-4-methylsulfonylanilin
N-Bis(n-propyl)-2,6-dinitro-4-aminosulfonylanilin
Bis(β-chloräthyl)-2,6-dinitro-4-methyl-anilin
N-Äthyl-N-(2-methylallyl)-2,6-dinitro-4-trifluormethyl-anilin
N-Methylcarbaminsäure-3,4-dichlorbenzylester
N-Methylcarbaminsäure-2,6-di(tert.-butyl)-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester

O-(N-Phenylcarbamoyl)-propanonoxim
N-Äthyl-2-(phenylcarbamoyl)-oxypropionsäureamid
3'-N-Isopropyl-carbamoyloxy-propionanilid
Äthyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-äthyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Äthyl-N-(3-(N'-3-chlor-4-fluorphenylcarbamoyloxy)-phenyl)-carbamat
Äthyl-N-(3-(N'-3,4-difluorphenylcarbamoyloxy)-phenyl)-carbamat
Methyl-N-(3-(N'-3,4-difluorphenylcarbamoyloxy)-phenyl)-carbamat
N-3-(4-Fluorphenoxycarbonylamino)-phenylcarbaminsäure-methylester
N-3-(2-Methylphenoxycarbonylamino)-phenylcarbaminsäureäthylester
N-3-(4-Fluorphenoxycarbonylamino)-phenylthiocarbaminsäuremethylester
N-3-(2,4,5-Trimethylphenoxycarbonylamino)-phenylthiolcarbaminsäure-methylester
N-3-(Phenoxycarbonylamino)-phenylthiolcarbaminsäure-methylester
N,N-Diäthyl-thiolcarbaminsäure-p-chlorbenzylester
N,N-Di-n-propyl-thiolcarbaminsäure-äthylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-äthyl-5-isoxazolyl-methylester
N,N-Di-sec.-butyl-thiolcarbaminsäure-äthylester
N,N-Di-sec.-butyl-thiolcarbaminsäure-benzylester
N-Äthyl-N-cyclohexyl-thiolcarbaminsäure-äthylester
N-Äthyl-N-bicyclo-[2,2,1]-heptyl-thiolcarbaminsäureäthylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Äthyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Äthyl-3-methylhexahydro-1-H-azepin-1-carbothiolat
N-Äthyl-N-n-butyl-thiolcarbaminsäure-n-propylester
N,N-Dimethyl-dithiocarbaminsäure-2-chlorallylester
N-Methyl-dithiocarbaminsäure-natrium
Trichloressigsäure-Na salz
α,α-Dichlorpropionsäure-Na salz
α,α-Dichlorbuttersäure-Na salz
α,α,β,β-Tetrafluorpropionsäure-Na salz
α-Methyl-α,β-dichlorpropionsäure-Na salz
α-Chlor-β-(4-chlorphenyl)-propionsäure-methylester
α,β-Dichlor-β-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureäthylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäuremethylester
2-[4-(2'-Chlor-4'-trifluorphenoxy)-phenoxy]-propionsäure-Na salz
2-[4-(3',5'-dichlorpyridyl-2-oxy)-phenoxy]-propionsäure-Na salz
2-(N-Benzoyl-3,4-dichlorphenylamino)-propionsäureäthylester
2-(N-Benzoyl-3-chlor-4-fluorphenylamino)-propionsäure-methylester
2-(N-Benzoyl-3-chlor-4-fluorphenylamino)-propionsäure-isopropylester
2-Chlor-4-äthylamino-6-isopropylamino-1,3,5-triazin
2-Chlor-4-äthylamino-6-(amino-2'-propionitril)-1,3,5-triazin
2-Chlor-4-äthylamino-6,2-methoxypropyl-2-amino-1,3,5-triazin
2-Chlor-4-äthylamino-6-butin-1-yl-2-amino-1,3,5-triazin
2-Chlor-4,6-bisäthylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-äthylamino-6-isopropylamino-1,3,5-triazin
2-Methylthio-4-äthylamino-6-tert.-butylamino-1,3,5-triazin
2-Methylthio-4,6-bisäthylamino-1,3,5-triazin
2-Methylthio-4,6-bisisopropylamino-1,3,5-triazin

2-Methoxy-4-äthylamino-6-isopropylamino-1,3,5-triazin
2-Methoxy-4,6-bisäthylamino-1,3,5-triazin
2-Methoxy-4,6-bisisopropylamino-1,3,5-triazin
4-Amino-6-tert.-butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.-butyl-3-methylthio-4,5-dihydro-1,2,4-triazin-5-on
1-Methyl-3-cyclohexyl-6-dimethylamino-1,3,5-triazin-2,4-dion
3-tert.-Butyl-5-chlor-6-methyluracil
3-tert.-Butyl-5-brom-6-methyluracil
3-Isopropyl-5-brom-6-methyluracil
3-sec.-Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]-äthan (Salze)
[-1-(1,2,4-Triazolyl-1')]-[1(4'-chlorphenoxy)]-3,3-dimethylbutan-2-on
N,N-Diallylchloracetamid
N-Isopropyl-2-chloracetanilid
N-(Butin-1-yl-3)-2-chloracetanilid
2-Methyl-6-äthyl-N-(propargyl)-2-chloracetanilid
2-Methyl-6-äthyl-N-(äthoxymethyl)-2-chloracetanilid
2-Methyl-6-äthyl-N-(2-methoxy-1-methyläthyl)-2-chloracetanilid
2-Methyl-6-äthyl-N-(isopropoxycarbonyläthyl)-2-chloracetanilid
2-Methyl-6-äthyl-N-(4-methoxypyrazol-1-yl-methyl)-2-chlor-acetanilid
2-Methyl-6-äthyl-N-(pyrazol-1-yl-methyl)-2-chloracetanilid
2,6-Dimethyl-N-(pyrazon-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxalan-2-yl-methyl)-2-chloracetanilid
2,6-Dimethyl-N-(2-methoxyäthyl)-2-chloracetanilid
2,6-Dimethyl-N-(isobutoxymethyl)-2-chloracetanilid
2,6-Diäthyl-N-(methoxymethyl)-2-chloracetanilid
2,6-Diäthyl-N-(n-butoxymethyl)-2-chloracetanilid
2,6-Diäthyl-N-(äthoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2-(2-Methyl)-4-chlorphenoxy-N-methoxy-acetamid
2-(α-Naphthoxy)-N,N-diäthylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-äthoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenyl-natriumsalz
2,4-Dichlorphenyl-4'-nitrophenyläther
2,4,6-Trichlorphenyl-4'-nitrophenyläther
2-Fluor-4,6-dichlorphenyl-4'-nitrophenyläther
2-Chlor-4-trifluormethylphenyl-4'-nitrophenyläther
2,4'-Dinitro-4-trifluormethyl-diphenyläther
2,4-Dichlorphenyl-3'-methoxy-4'-nitro-phenyläther
2-Chlor-4-trifluormethylphenyl-3'-äthoxy-4'-nitro-phenyläther
2-Chlor-4-trifluormethylphenyl-3'-carboxy-4'-nitro-phenyläther (Salze)
2-Chlor-4-trifluormethylphenyl-3'-äthoxycarbonyl-4'-nitrophenyläther
2,4-Dichlorphenyl-3'-methoxycarbonyl-4'-nitrophenyläther
2-(3,4-Dichlorphenyl)-4-methyl-1,2,4-oxadiazolidin-3,5-dion
2-(3-tert.-Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$.$0^{8,11}$]-dodeca-3,9-dien
2-Äthoxy-2,3-dihydro-3,3-dimethyl-5-benzofuranyl-methansulfonat
2-Äthoxy-2,3-dinhydro-3,3-dimethyl-5-benzofuranyl-dimethyl-aminosulfonat

2-Äthoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.-Butyl-4,6-dinitrophenol (Salze, Ester)
2-sec.-Butyl-4,6-dinitrophenol-acetat
2-tert.-Butyl-4,6-dinitrophenol-acetat
2-tert.-Butyl-4,6-dinitrophenol (Salze)
2-tert.-Butyl-5-methyl-4,6-dinitrophenol (Salze)
2-tert.-Butyl-5-methyl-4,6-dinitrophenol-acetat
2-sec.-Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3.3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(α,α,β,β-Tetrafluoräthoxyphenyl)-3,3-dimethyl-harnstoff
1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4-(4'-Chlorphenoxy)-phenyl]-3,3-dimethyl-harnstoff
1-[4-(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.-Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
1-(4-Benzyloxyphenyl)-3-methyl-3-methoxy-harnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di(methylsulfat)
1,1'-Di-(3,5-dimethylmorpholin-carbonylmethyl)-4,4'-dipyridylium-dichlorid
1,1'-Äthylen-2,2'-dipyridylium-dibromid
3-[1-(N-Äthoxyamino)-propyliden]-6-äthyl-3,4-dihydro-2-H-pyran-2,4-dion
3-[1-(N-Allyloxyamino)-propyliden]-6-äthyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)

4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chloräthanphosphonsäure-2-chloräthylester
Ammonium-äthyl-carbamoyl-phosphonat
Di-n-butyl-1-n-butylamino-cyclohexyl-phosphonat
Trithiobutylphosphit
O,O-Diisopropyl-5-(2-benzosulfonylamino-äthyl)-phosphordithionat
2,3-Dihydro-5,6-dimethyl-1,4-dithiin-1,1,4,4-tetraoxid
5-tert.-Butyl-3-(2,4-dichlor-5-isopropoxy-phenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chloräthyl)-trimethyl-ammoniumchlorid
(2-Methyl-4-phenylsulfonyl)-trifluormethan-sulfonanilid
1,1-Dimethyl-4,6-diisopropyl-5-indanyläthyl-keton
2-[1-(2,5-Dimethylphenyl)-äthylsulfonyl]-pyridin-N-oxid
Natriumchlorat
Ammoniumrhodanid
Calciumcyanamid

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können Netz- und Haftmittel sowie nicht-phytotoxische Öle und Ölkonzentrate zugesetzt werden.

Der Einfluss verschiedener Vertreter der erfindungsgemässen Verbindungen auf das Wachstum von unerwünschten und erwünschten Pflanzen wird durch Gewächshausversuche gezeigt:

Als Kulturgefässe dienten Plastikblumentöpfe mit 300 $cm^3$ Inhalt und lehmigem Sand mit etwa 1,5% Humus als Substrat. Die Samen der Testpflanzen entsprechend Tabelle 1 wurden, nach Arten getrennt, flach eingesät. Bei *Cyperus esculentus* wurden vorgekeimte Knollen genommen.

Bei Vorauflaufbehandlung wurden die Wirkstoffe unmittelbar danach auf die Erdoberfläche aufgebracht. Sie wurden hierbei in Wasser als Verteilungsmittel suspendiert oder emulgiert und mittels fein verteilenden Düsen gespritzt. Nach dem Aufbringen der Mittel wurden die Gefässe leicht beregnet, um Keimung und Wachstum in Gang zu bringen und die chemischen Mittel zu aktivieren. Dann wurden die Gefässe mit durchsichtigen Plastikhauben abgedeckt, bis die Pflanzen angewachsen waren. Diese Abdeckung bewirkte ein gleichmässiges Keimen der Testpflanzen, sofern dies nicht durch die Chemikalien beeinträchtigt wurde.

Zum Zwecke der Nachauflaufbehandlung zog man die Pflanzen je nach Wuchsform in den Versuchsgefässen erst bis zu einer Höhe von 3 bis 10 cm an und behandelte sie danach. Einige Pflanzenarten waren dabei direkt in die zu behandelnden Töpfe gesät, andere wurden in Anzuchtschalen vorgezogen und einige Tage vor der Behandlung in die Versuchsgefässe transplantiert. Die der Nachauflaufbehandlung unterzogenen Pflanzen wurden nicht abgedeckt.

Die Versuchsgefässe wurden im Gewächshaus aufgestellt, wobei für wärmeliebende Arten wärmere Bereiche von 25 bis 40°C und für solche gemässigter Klimate Temperaturen von 15 bis 30°C bevorzugt wurden. Die Versuchsperiode erstreckte sich über 2 bis 4 Wochen. Während dieser Zeit wurden die Pflanzen gepflegt, und ihre Reaktion auf die einzelnen Behandlungen wurde ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Sprossteile.

Die in den Versuchen getesteten Pflanzenarten sind in Tabelle 1 aufgeführt.

Die Tabellen 2 bis 10 zeigen die selektive herbizide Wirkung von Vertretern der erfindungsgemässen Verbindungen. Diese richtet sich je nach Verbindung entweder spezifisch gegen einzelne breitblättrige unerwünschte Pflanzen oder gleichzeitig gegen breitblättrige und grasartige unerwünschte Pflanzen; die Wirkung umfasst auch Cyperaceen. Die erfindungsgemässen Mittel können sowohl im Vor- als auch im Nachauflaufverfahren eingesetzt werden; bevorzugt ist jedoch die Nachauflaufanwendung. Eine besondere Ausbringungstechnik besteht darin, dass die Wirkstoffe mit Hilfe der Spitzgeräte so gespritzt werden, dass die Blätter empfindlicher Kulturpflanzen nach Möglichkeit nicht getroffen werden, während die Wirkstoffe auf die darunterliegende Bodenfläche oder dort wachsende unerwünschte Pflanzen gelangen (post-directed, lay-by).

Tabelle 1 – Liste der Pflanzennamen

| Botanischer Name (Abk. in Tabelle) | Deutscher Name | Englischer Name |
|---|---|---|
| *Abutilon theophrasti* | Chinesischer Hanf | velvet leaf |
| *Amaranthus retroflexus* | zurückgekrümmter Fuchsschwanz | redroot pigweed |

| Botanischer Name (Abk. in Tabelle) | Deutscher Name | Englischer Name |
|---|---|---|
| *Arachis hypogaea* | Erdnuss | peanuts (groundnuts) |
| *Beta vulgaris* | Zuckerrübe | sugarbeets |
| *Bromus spp.* | Trespearten | broome |
| *Bidens pilosa* | Zweizahn | hairy beggarticks |
| *Centaurea cyanus* | Kornblume | cornflower |
| *Chenopodium album* | Weisser Gänsefuss | lambsquarters (goosefoot) |
| *Chrysanthemum segetum* | Saatwucherblume | corn marigold |
| *Cyperus esculentus* | Erdmandel | yellow nutsedge |
| *Cyperus ferax* | | |
| *Datura stramonium* | Gemeiner Stechapfel | Jimsonweed |
| *Digitaria sanguinalis* | Blutfingerhirse | large crabgrass |
| *Euphoria geniculata* | Südamerik. Wolfsmilchart | Southamerican member of the spurge family |
| *Glycine max* | Sojabohnen | soybeans |
| *Helianthus annuus* | Sonnenblume | sunflowers |
| *Ipomoea spp.* | Prunkwindearten | morningglory |
| *Nicandra physaloides* | Giftbeere | apple-of-Peru |
| *Oryza sativa* | Reis | rice |
| *Pisum sativum* | Erbsen | English peas |
| *Polygonum persicaria* | Flohknöterich | ladystumb |
| *Sesbania exaltata* | Turibaum | hemp sesbania (coffeeweed) |
| *Setaria spp.* | Borstenhirsearten | foxtail spp. |
| *Asclepias syriaca* | | milkweed |
| *Sinapis alba* | Weisser Senf | white mustard |
| *Solanum nigrum* | Schwarzer Nachtschatten | black nightshade |
| *Triticum aestivum* | Weizen | wheat |
| *Zea mays* | Mais | Indian corn |
| *Acanthospermum hispidum* | — | bristly starbur |
| *Asclepias spp.* | Seidenpflanze | milkweed |
| *Avena fatua* | Flughafer | wild oats |
| *Centaurea cyanus* | Kornblume | cornflower |
| *Hordeum vulgare* | Gerste | barley |
| *Lamium amplexicaule* | stengelumfassende Taubnessel | henbit |
| *Mercurialis annua* | einjähriges Bingelkraut | annual mercury |
| *Viola spp.* | Stiefmütterchen | pansy |

*Tabelle 2*

Selektive Beseitigung von Melden in Zuckerrüben und anderen Kulturen bei Nachauflaufanwendung im Gewächshaus mit dem Wirkstoff der Formel

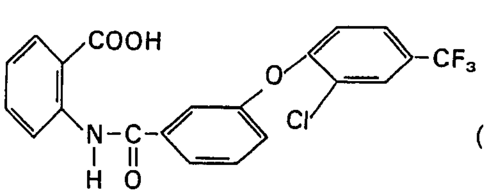

(Nr. 12)

| Testpflanzen | Schädigung [%] bei Aufwandmengen von 1,0 | 0,4 kg a.S./ha |
|---|---|---|
| *Beta vulgaris* | 0 | 0 |
| *Helianthus annuus* | 0 | 0 |
| *Pisum sativum* | 0 | 0 |
| *Chenopodium album* | 99 | 99 |

*Tabelle 3*

Selektive Bekämpfung von Amaranth in Weizen und Zuckerrüben bei Nachauflaufanwendung im Gewächshaus mit dem Wirkstoff der Formel

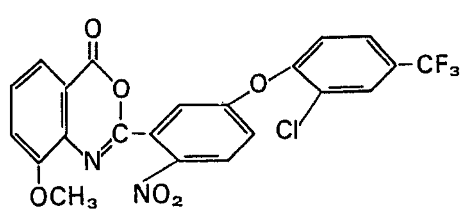

(Nr. 50)

Vergleichsmittel ist der Wirkstoff der Formel

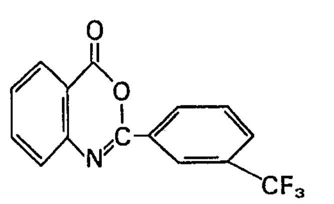

(US-PS 3914121)

| Testpflanzen | Schädigung [%] bei Aufwandmengen von | |
|---|---|---|
| | 0,5 kg Wirkstoff Nr. 50/ha | 0,5 kg Vergleichsmittel/ha |
| *Beta vulgaris* | 10 | 95 |
| *Triticum aestivum* | 10 | 0 |
| *Amaranthus retroflexus* | 90 | 16 |

*Tabelle 4*

Beseitigung von unerwünschten Gräsern und Cyperus in verschiedenen Kulturen bei Nachauflaufanwendung im Gewächshaus mit dem Wirkstoff der Formel

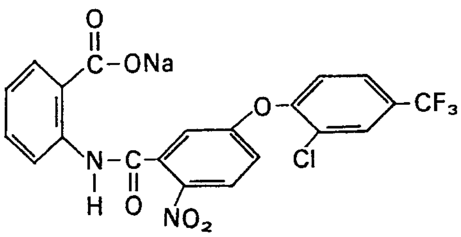

(Nr. 4)

| Testpflanzen | Schädigung [%] bei einer Aufwandmenge von 0,4 kg a.S./ha |
|---|---|
| *Arachis hypogaea* | 0 |
| *Glycine max* | 12 |
| *Oryza sativa* | 10 |
| *Pisum sativum* | 0 |
| *Zea mays* | 10 |
| *Cyperus esculentus* | 80 |
| *Setaria spp.* | 80 |

*Tabelle 5*

Bekämpfung von Blutfingerhirse in Soja und Reis bei Nachauflaufanwendung im Gewächshaus mit dem Wirkstoff der Formel

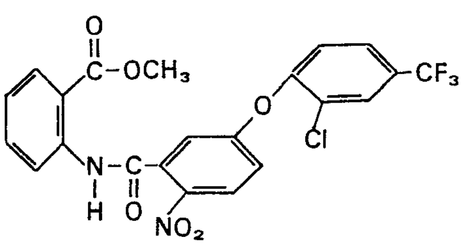

(Nr. 1)

| Testpflanzen | Schädigung [%] bei einer Aufwandmenge von 0,5 kg a.S./ha |
|---|---|
| *Glycine max* | 0 |
| *Oryza sativa* | 0 |
| *Digitaria sanguinalis* | 80 |

*Tabelle 6*

Bekämpfung von *Cyperus spp.* in Erdnüssen und Reis bei Nachauflaufanwendung im Gewächshaus mit dem Wirkstoff der Formel

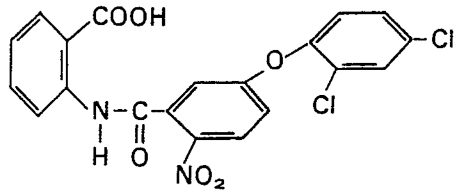

(Nr. 7)

| Testpflanzen | Schädigung [%] bei Aufwandmengen von | |
|---|---|---|
| | 0,5 | 0,25 kg a.S./ha |
| *Arachis hypogaea* | 0 | — |
| *Oryza sativa* | 10 | 0 |
| *Cyperus ferax* | 100 | 100 |

*Tabelle 7*

Selektive Bekämpfung von breitblättrigen Unkräutern in Erdnüssen bei Nachauflaufanwendung im Gewächshaus mit dem Wirkstoff der Formel

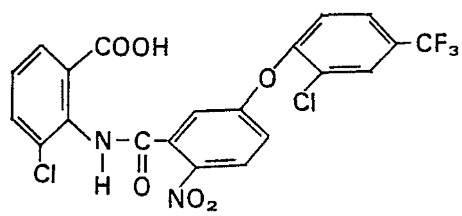

(Nr. 23)

| Testpflanzen | Schädigung [%] bei einer Aufwandmenge von 0,4 kg a.S./ha |
|---|---|
| *Arachis hypogaea* | 0 |
| *Euphorbia geniculata* | 85 |
| *Solanum nigrum* | 80 |

*Tabelle 8*

Bekämpfung unerwünschter Pflanzen in verschiedenen landwirtschaftlichen Kulturen bei Nachauflaufanwendung im Gewächshaus mit dem Wirkstoff der Formel

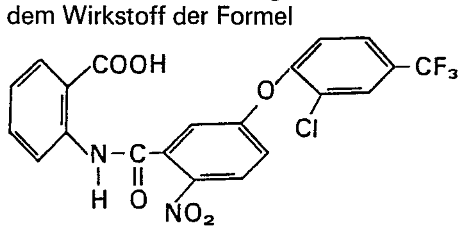

(Nr. 2)

Vergleichsmittel ist der Wirkstoff der Formel

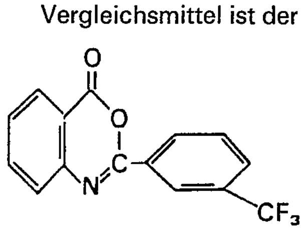

(US-PS 3914121)

| Testpflanzen | Schädigung [%] bei einer Aufwandmenge von 0,25 kg Wirkst. Nr. 2/ha | 0,25 kg Vergleichsm./ha |
|---|---|---|
| *Arachis hypogaea* | 10 | 0 |
| *Glycine max* | 10 | 22 |
| *Oryza sativa* | 2 | 0 |
| *Pisum sativum* | 10 | 2 |
| *Abutilon theophrasti* | 100 | 10 |
| *Bromus spp.* | 80 | 0 |
| *Chrysanthemum segetum* | 100 | 82 |
| *Cyperus ferax* | 99 | 30 |
| *Digitaria sanguinalis* | 90 | 0 |
| *Polygonum persicaria* | 98 | 60 |
| *Sesbania exaltata* | 92 | 90 |
| *Setaria spp.* | 90 | 0 |

*Tabelle 9*

Bekämpfung unerwünschten Pflanzenwuchses bei Nachauflaufanwendung im Gewächshaus mit dem Wirkstoff der Formel

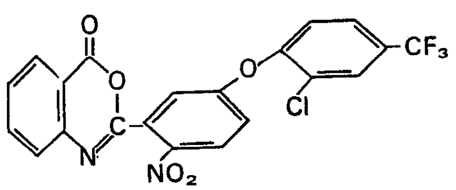

(Nr. 5)

Vergleichsmittel ist der Wirkstoff der Formel

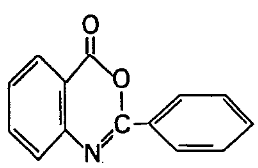

(DE-PS 1 191 171)

| Testpflanzen | Schädigung [%] bei einer Aufwandmenge von 0,4 kg Wirkst. Nr. 5/ha | 4,0 kg Vergleichsm./ha |
|---|---|---|
| *Arachis hypogaea* | 5 | 0 |
| *Abutilon theophrasti* | 98 | 10 |
| *Asclepias syriaca* | 90 | 0 |
| *Bidens pilosa* | 95 | 20 |
| *Datura stramonium* | 85 | 10 |
| *Digitaria sanguinalis* | 90 | — |
| *Euphorbia geniculata* | 98 | 20 |
| *Nicandra physaloides* | 100 | 98 |
| *Setaria spp.* | 80 | 0 |
| *Sinapis alba* | 95 | 95 |
| *Solanum nigrum* | 100 | 65 |

*Tabelle 10*

Herbizide Wirkung bei Vor- und Nachauflaufanwendung im Gewächshaus

| Wirkstoff Nr. | Aufwandmenge [kg a.S./ha] | Testpflanzen und Schädigung [%] Vorauflauf *Sinapis alba* | Nachauflauf *Centaurea cyanus* | *Ipomoea spp.* |
|---|---|---|---|---|
| 21 | 3,0 | 100 | 90 | — |
| 15 | 3,0 | 100 | 90 | 90 |
| 29 | 3,0 | 90 | 90 | — |
| 37 | 3,0 | 100 | 100 | 100 |
| 30 | 3,0 | 90 | 100 | — |
| 55 | 3,0 | — | 90 | 80 |
| 45 | 3,0 | 100 | 100 | 100 |
| 51 | 3,0 | 90 | 100 | 100 |
| 38 | 3,0 | 100 | 100 | 100 |

*Tabelle 11*

Selektive Bekämpfung unerwünschter breitblättriger Pflanzen in Gramineenkulturen bei Nachauflaufanwendung im Gewächshaus mit dem Wirkstoff der Formel

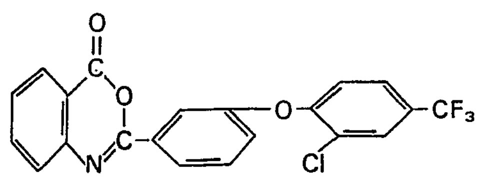

(Nr. 49)

| Testpflanzen | Schädigung [%] bei kg/ha a.S. 1,0 | 2,0 |
|---|---|---|
| *Hordeum vulgare* | 0 | 0 |
| *Oryza sativa* | 10 | 10 |
| *Triticum aestivum* | 0 | 0 |
| *Acanthospermum hispidum* | 100 | 100 |
| *Asclepias spp.* | 98 | 100 |
| *Chenopodium album* | 100 | 100 |
| *Chrysanthemum segetum* | 100 | 100 |
| *Euphorbia geniculata* | 100 | 100 |
| *Lamium amplexicaule* | 80 | 80 |
| *Nicandra physaloides* | 100 | 100 |
| *Solanum nigrum* | 75 | 75 |
| *Sinapis alba* | 80 | 100 |

0 = keine Schädigung
100 = Pflanzen völlig abgestorben

*Tabelle 12*

Selektive Bekämpfung unerwünschter Pflanzen bei Nachauflaufanwendung im Gewächshaus mit dem Wirkstoff der Formel

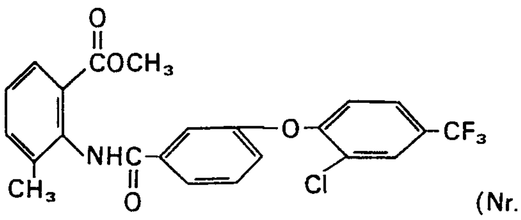

(Nr. 19)

| Testpflanzen | Schädigung [%] bei 0,5 kg/ha a.S. |
|---|---|
| *Triticum aestivum* | 5 |
| *Amaranthus retroflexus* | 100 |
| *Avena fatua* | 80 |
| *Ipomoea spp.* | 95 |

0 = ohne Schädigung
100 = Pflanzen völlig abgestorben

*Tabelle 13*

Bekämpfung von breitblättrigen unerwünschten Pflanzen in Getreide bei Nachauflaufanwendung im Gewächshaus mit dem Wirkstoff der Formel

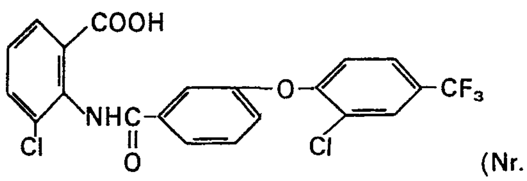

(Nr. 22)

| Testpflanzen | Schädigung [%] bei kg/ha a.S. 1,0 | 2,0 |
|---|---|---|
| *Triticum aestivum* | 5 | 5 |
| *Centaurea cyanus* | 98 | 98 |
| *Ipomoea spp.* | 100 | 100 |
| *Mercurialis annua* | 95 | 95 |
| *Sinapis alba* | 100 | 100 |
| *Viola spp.* | 80 | 90 |

0 = ohne Schädigung
100 = Pflanzen völlig abgestorben

**Patentansprüche**

1. Substituierte N-Benzoylanthranilsäurederivate der Formel I:

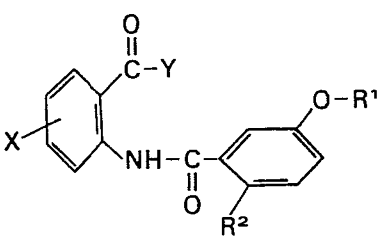

(I)

und deren Anhydroverbindungen der Formel II:

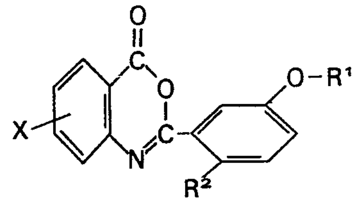

(II)

in denen

$R^1$ für einen substituierten Phenylrest der Formel:

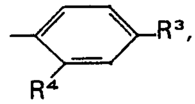

wobei $R^3$ und $R^4$ jeweils unabhängig voneinander Halogen oder Halogenalkyl mit 1 bis 4 Kohlenstoffatomen bedeuten,

$R^2$ für Wasserstoff oder Nitro,

X für Wasserstoff, Halogen, Alkyl oder Alkoxy mit jeweils 1 bis 4 Kohlenstoffatomen und

Y für den Rest $-OR^6$, in dem $R^6$ Wasserstoff, ein Alkalimetallkation oder Alkyl mit bis zu 4 Kohlenstoffatomen bedeutet, stehen.

2. Natriumsalz der N-3'-(2"-Chlor-4"-trifluormethyl-phenoxy)-6'-nitrobenzoylanthranilsäure.

3. 2-[3'-(2"-Chlor-4"-trifluormethyl-phenoxy)-6'-nitro-phenyl]-4H-1,3-benzoxazin-4-on.

4. N-3'-(2"-Chlor-4"-trifluormethyl-phenoxy)-benzoylanthranilsäure.

5. Herbizid, enthaltend mindestens ein N-Benzoylanthranilsäurederivat der Formel I und/oder eine Anhydroverbindung der Formel II gemäss Anspruch 1.

6. Verfahren zur Bekämpfung unerwünschten Pflanzenwuchses, dadurch gekennzeichnet, dass man ein Herbizid, enthaltend mindestens ein N-Benzoylanthranilsäurederivat der Formel I und/oder eine Anhydroverbindung der Formel II gemäss Anspruch 1, verwendet.

7. Verfahren zur Herstellung von N-Benzoylanthranilsäurederivaten der Formel I, dadurch gekennzeichnet, dass man Anthranilsäurederivate der Formel III:

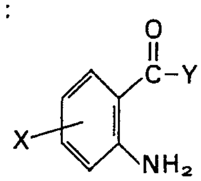

(III)

in der X und Y die im Anspruch 1 genannten Bedeutungen haben, mit ungefähr stöchiometrischen Mengen eines substituierten Benzoylhalogenids der Formel IV:

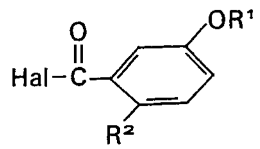

(IV)

in der $R^1$ und $R^2$ die im Anspruch 1 genannten Bedeutungen haben und Hal für Halogen steht, in wässerigem alkalischem Medium oder gegebenenfalls in Gegenwart eines säurebindenden Mittels in einem inerten organischen Lösungsmittel

bei einer Temperatur im Bereich zwischen −30 und +150°C umsetzt.

8. Verfahren zur Herstellung von Verbindungen der Formel II, dadurch gekennzeichnet, dass man Anthranilsäurederivate der Formel III:

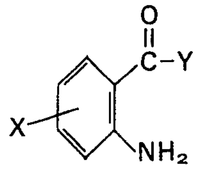
(III)

in der X und Y die im Anspruch 1 genannten Bedeutungen haben, mit mindestens dem zweifachen molaren Überschuss eines substituierten Benzoylhalogenids der Formel IV:

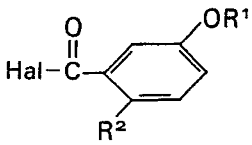
(IV)

in der $R^1$ und $R^2$ die im Anspruch 1 genannten Bedeutungen haben und Hal für Halogen steht, in Gegenwart eines aromatischen tertiären Amins bei einer Temperatur im Bereich zwischen 0 und 150°C umsetzt.

9. Verfahren zur Herstellung von Verbindungen der Formel II, dadurch gekennzeichnet, dass man Anthranilsäurederivate der Formel V:

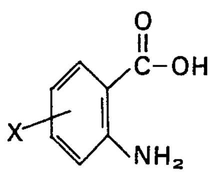
(V)

in der X die im Anspruch 1 genannten Bedeutungen hat mit ungefähr stöchiometrischen Mengen eines substituierten Benzoylhalogenids der Formel IV:

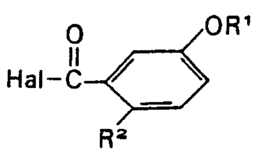
(IV)

in der $R^1$ und $R^2$ die im Anspruch 1 genannten Bedeutungen haben und Hal für Halogen steht, in wässerigem alkalischem Medium oder gegebenenfalls in Gegenwart eines säurebindenden Mittels in einem inerten organischen Lösungsmittel bei einer Temperatur im Bereich zwischen −30 und +150°C zu einem N-Benzoylanthranilsäurederivat der Formel I umsetzt und dieses dann in Gegenwart eines wasserentziehenden Mittels bei einer Temperatur im Bereich zwischen 0 und 150°C cyclisiert.

## Claims

Substituted N-benzoylanthranilic acid derivates of the formula I:

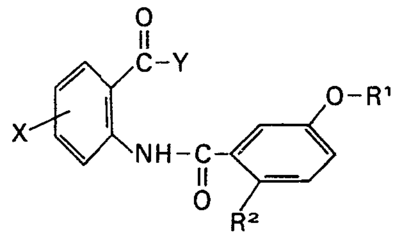
(I)

and their anhydro compounds, of the formula II

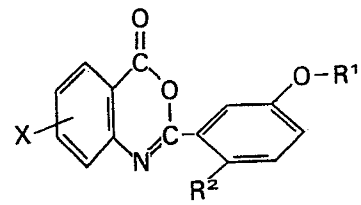
(II)

where

$R^1$ is a substituted phenyl of the formula

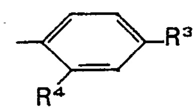

where $R^3$, $R^4$ are each, independently of one another, halogen or haloalkyl of 1 to 4 carbon atoms,

$R^2$ is hydrogen or nitro,

X is hydrogen, halogen, alkyl or alkoxy each of 1 to 4 carbon atoms, and

Y is $-OR^6$ where $R^6$ is hydrogen, an alkali metal cation or alkyl of up to 4 carbon atoms.

2. The sodium salt of N-3′-(2″-chloro-4″-trifluormethylphenoxy)-6′-nitrobenzoylanthranilic acid.

3. 2-[3′-(2″-Chloro-4″-trifluormethylphenoxy)-6′-nitro-phenyl]-4H-1,3-benzoxazin-4-one.

4. N-3′-2″-Chloro-4″-trifluormethylphenoxy)-benzoyl-anthranilic acid.

5. A herbicide containing at least one N-benzoylanthranilic acid derivate of the formula I, and/or an anhydro compound of the formula II, as claimed in claim 1.

6. A process for combating the growth of unwanted plants, wherein a herbicide is employed which contains at least one N-benzoylanthranilic acid derivate of the formula I, and/or an anhydro compound of the formula II, as claimed in claim 1.

7. A process for the production of N-benzoylanthranilic acid derivates of the formula I wherein anthranilic acid derivates of the formula III:

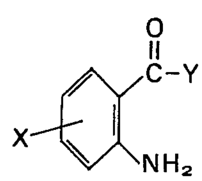
(III)

where X and Y have the meanings given in claim 1, are reacted with about stoichiometric amounts of a substituted benzoyl halide of the formula IV:

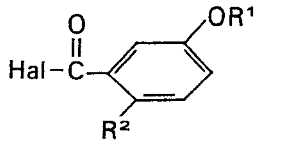

(IV)

where $R^1$ and $R^2$ have the meanings given in claim 1 and Hal is halogen, in a aqueous alkaline medium or,
where appropriate, in the presence of an acid-binding agent in an inert organic solvent at from −30 to +150°C.

8. A process for the production of compounds of the formula II wherein anthranilic acid derivates of the formula III:

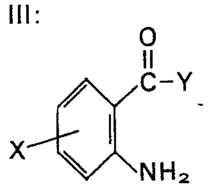

(III)

where X and Y have the meanings given in claim 1, are reacted with at least twice the molar excess of a substituted benzoyl halide of the formula IV:

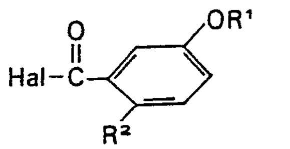

(IV)

where $R^1$ and $R^2$ have the meanings given in claim 1 and Hal is halogen, in the presence of an aromatic tertiary amine at between 0 and 150°C.

9. A process for the preparation of compounds of the formula II wherein anthranilic acid derivates of the formula V:

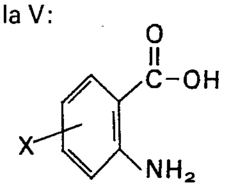

(V)

where X as the meanings given in claim 1, are reacted with approximately stoichiometric amounts of a substituted benzoyl halide of the formula IV:

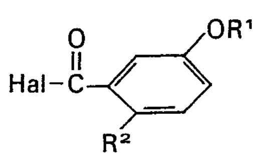

(IV)

where $R^1$ and $R^2$ have the meanings given in claim 1, and Hal is halogen, in an aqueous alkaline medium or, if desired, in the presence of an acid-binding agent in an inert organic solvent at a temperature in the range of from −30 to +150°C to give an N-benzoylanthranilic acid derivate of the formula I and the latter is then cyclized in the presence of a dehydrating agent at a temperature in the range between 0 and 150°C.

**Revendications**

1. Dérivés substitués d'acide N-benzoyl-anthranilique de formule I:

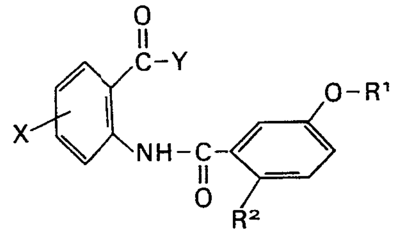

(I)

et leurs composés anhydro de formule II:

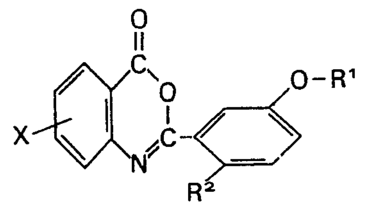

(II)

dans lesquelles

$R^1$ représente un reste phényle substitué de formule

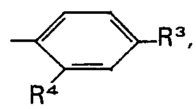

$R^3$ et $R^4$ étant chacun, indépendamment l'un de l'autre, un halogène ou halogénalkyle à 1 à 4 atomes de carbone,

$R^2$ représente hydrogène ou nitro,

X hydrogène, halogène, alkyle ou alcoxy ayant chacun 1 à 4 atomes de carbone, et

Y le reste $-OR^6$, où $R^6$ représente hydrogène, un cation métal alcalin ou un alkyle ayant jusqu'à 4 atomes de carbone.

2. Sel de sodium de l'acide N-3'-(2"-chloro-4"-trifluorométhyl-phénoxy)-6'-nitrobenzoyl-anthranilique.

3. 2-[3'-(2"-chloro-4"-trifluorométhyl-phénoxy)-6'-nitro-phényl]-4H-1,3-benzoxazin-4-one.

4. Acide N-3'-(2"-chloro-4"-trifluorométhyl-phénoxy)-benzoylanthranilique.

5. Herbicide contenant au moins un dérivé d'acide N-benzoylanthranilique de formule I et/ou un composé anhydro de formule II selon la revendication 1.

6. Procédé de lutte contre la croissance indésirable des plantes, caractérisé par le fait qu'on utilise un herbicide contenant au moins un dérivé d'acide N-benzoylanthranilique de formule I et/ou un composé anhydro de formule II selon la revendication 1.

7. Procédé de préparation de dérivés d'acide N-benzoylanthranilique de formule I, caractérisé par le fait qu'à une température comprise entre −30 et +150C, en milieu alcalin aqueux ou éventuellement en présence d'un agent liant les acides, dans un solvant organique inerte, on fait réagir un dérivé d'acide anthranilique de formule III:

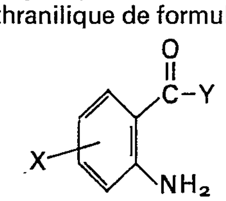

(III)

dans laquelle X et Y ont les significations indiquées dans la revendication 1, avec des quantités à peu près stœchiométriques d'un halogénure de benzoyle substitué de formule IV:

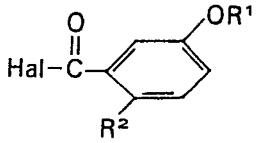

(IV)

dans laquelle $R^1$ et $R^2$ ont les significations indiquées dans la revendication 1 et Hal représente un halogène.

8. Procédé de préparation de composés de formule II, caractérisé par le fait qu'à une température comprise entre 0 et 150°C, en présence d'une amine tertiaire aromatique, on fait réagir des dérivés d'acide anthranilique de formule III:

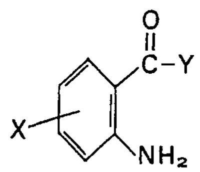

(III)

dans laquelle X et Y ont les significations indiquées dans la revendication 1, avec au moins le double excès molaire d'un halogénure de benzoyle substitué de formule IV:

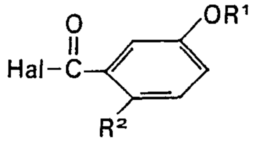

(IV)

dans laquelle $R^1$ et $R^2$ ont les significations indiquées dans la revendication 1 et Hal représente un halogène.

9. Procédé de préparation de composés de formule II, caractérisé par le fait qu'à une température comprise entre −30 et +150°C, en milieu alcalin aqueux ou éventuellement en présence d'un agent liant les acides, dans un solvant organique inerte, on fait réagir un dérivé d'acide anthranilique de formule V:

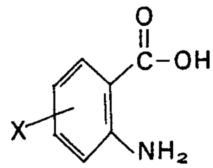

(V)

dans laquelle X a la signification indiquée dans la revendication 1, avec des quantités à peu près stoéchiométriques d'un halogénure de benzoyle substitué de formule IV:

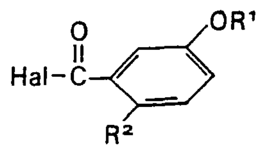

(IV)

dans laquelle $R^1$ et $R^2$ ont les significations indiquées dans la revendication 1 et Hal représente un halogène pour obtenir un dérivé d'acide N-benzoylanthranilique de formule I qu'on cyclise ensuite en présence d'un agent déshydratant à une température comprise entre 0 et 150°C.